# EUROPEAN PATENT SPECIFICATION

(11) **EP 3 831 809 B1**
(45) Date of publication and mention of the grant of the patent: **13.09.2023**
(21) Application number: 20211599.4
(22) Date of filing: 03.12.2020
(51) Int. Cl.: C07C 273/00, F04D 7/06, F04D 1/06, F04D 31/00

(54) **CENTRIFUGAL PUMP FOR PROCESSING MOLTEN UREA AND RELATIVE PLANT**
KREISELPUMPE ZUR VERARBEITUNG VON GESCHMOLZENEM HARNSTOFF UND ENTSPRECHENDE ANLAGE
POMPE CENTRIFUGE POUR LE TRAITEMENT D'URÉE FONDUE ET INSTALLATION RELATIVE

(30) Priority: 06.12.2019 IT 201900023160
(43) Date of publication of application: 09.06.2021
(73) Proprietor: EUROTECNICA MELAMINE AG, 8832 Wollerau (CH)
(72) Inventor: SANTUCCI, Roberto, 21050 Gorla Maggiore (VA) (IT); DI RUOCCO, Giuseppe, 23900 Lecco (IT); DE AMICIS, Alberto, 20097 San Donato Milanese (MI) (IT)
(74) Representative: De Gregori, Antonella

(56) References cited:
- CN-A- 109 404 292
- CN-A- 109 723 618
- CN-U- 202 560 651
- US-A- 3 118 386
- US-A1- 2005 056 147
- US-A1- 2017 191 480
- US-A1- 2019 211 833

## Description

### FIELD OF THE INVENTION

The present invention relates to the field of industrial processing of urea, particularly for the production of melamine. In more detail, the present invention relates to a centrifugal pump for processing molten urea and a relative plant, in particular for producing melamine with a high-pressure process.

### STATE OF THE ART

The production of melamine by urea pyrolysis according to the global reaction (1) is known: 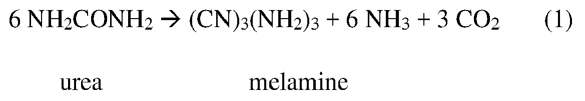 such reaction, as known, being highly endothermic.

The processes for converting urea into melamine are distinguished into two groups: processes performing high-pressure urea pyrolysis and processes performing low-pressure urea pyrolysis.

Both the processes are typically performed in reactors fed with a molten urea stream. Preferably, the reactor is also fed with an ammonia stream.

In high-pressure processes, the reaction chamber is always held at a pressure greater than 60 bar and it is provided with heating means maintaining the reagent system at a temperature of about 360°C - 450°C.

The present invention relates particularly to processes for the high-pressure preparation of melamine. More specifically, in order to produce melamine, it is necessary to handle and pressurize liquid-state urea with a concentration greater than 99.8%, i.e. molten, taking it either from a vacuum separator, in case the urea is fed to the plant as aqueous solution and needs to be concentrated inside the plant itself, or from a surge drum maintained under a slight pressure, in case the urea is fed to the melamine plant already as concentrated, to a reactor operating at a pressure of about 80 bars.

At present, in order to pressurize the molten urea from the separator to the reactor reciprocating pumps are used. The constant increase of the production capability of plants for producing melamine requested by the market, has determined a proportional increase of urea flow rates to be processed in the plants, resulting in an increase of the volume and number of reciprocating pump heads used, and frequently, in the use of more pumps in parallel, with the result of an increase in the complexity, costs and malfunction risks of the reciprocating pumps currently used with an impact on the operation of the plant itself. The use of reciprocating pumps causes a number of disadvantages.

Reciprocating pumps are by their nature in each head equipped with valves at the inlet (suction) and at the outlet (delivery) that control their operation. The presence of such valves along the pump's suction flow, and in particular at the inlet on each head, is a bottleneck that results in an speed increase and load loss with a subsequent decrease inside the valve itself causing the release of any gases dissolved in the liquid phase with a risk of cavitation phenomena at the pump inlet.

The molten urea is subjected to a continuous, though minimal, decomposition with the formation of ammonia and carbon dioxide, that remain dissolved in the liquid phase under constant pressure conditions, but which are released as a gas phase as the pressure decreases, generating cavitation phenomena that impair the proper operation of the reciprocating pumps reducing their performance and often resulting in mechanical faults. For this reason, reciprocating pumps require the use of an additional pump arranged upstream to cause a pre-compression of the molten urea and preventing cavitation at the reciprocating pump inlet.

Furthermore, the reciprocating pumps have by their nature a pulsating delivery pressure, which requires the use of accumulators downstream the reciprocating pump and other devices capable of damping pulsations and/or operating under pulsating pressures. Moreover, in the production of melamine, this factor affects the reactor subjected to a pulsating feeding which generates the formation of a pulsating reaction gas (see the reaction) with a subsequent internal pulsating pressure and on gases it emits in the pressure control, that also result having a pulsating flow rate, and on the components designated to processing such gases.

Urea has a melting temperature equal to 133 °C. In order to avoid urea solidification, the pump needs to be drained and washed after its shutdown. The reciprocating pumps with multiple heads arranged in parallel require that each head to be drained and washed individually, and therefore they require complex draining and washing systems. Such systems are detrimental to the reliability of the pump, increasing the possibility of leaks and clogging, and making these operations particularly time-consuming for the operators who could omit complete execution of the same.

Moreover, the starting of the reciprocating pumps causes a sudden pressure increase in the delivery of the pump itself, which risks to cause malfunctions or damages to the downstream components, particularly to the shut off/check valve, which keeps the reactor under pressure during the urea pre-feeding step wherein the pressure of the reactor is maintained by a constant supply of ammonia. In order to overcome this problem generally the delivery is pre-pressurized in advance, with a consequent increase in the complexity of the plant and of its starting procedures.

In addition, some plants require the presence of spare pumps arranged in parallel to the main pump and kept in stand-by, ready to be started if the running pump will stop. In order to maintain the spare pump in stand-by it is necessary to keep it empty and continuously flushed with steam to ensure that internally, in case of leakage of the shutoff valves, urea cannot accumulate, and with its subsequent degradation resulting in a blockage. As an alternative, it is necessary to pre-fill the stand-by pump with molten urea, guaranteeing a circulation inside it to avoid solidifications. In reciprocating pumps, establishing a recirculation inside the pump is complicated and difficult to obtain in a uniformly distributed manner due to the multiple heads which are not individually flushed, unless a complex circulation system is created for each individual head, thus resulting in increased operational operations and a high number of possible leakage points of urea outwards.

The document CN 109 404 292 A discloses a centrifugal pump according to the preamble of claim 1.

### SUMMARY

A general object of the present invention is to improve the prior art with reference to one or more points of view.

Particularly, an object of the present invention is to overcome the above disadvantages by using a centrifugal pump for processing molten urea and a relative plant, in particular for producing melamine with a high-pressure process.

More in detail, object of the present invention is to provide a centrifugal pump for processing molten urea and a relative plant capable of processing a higher amount of molten urea than the prior art, particularly a centrifugal pump having the prevalence conditions necessary to feed molten urea to the production reactor.

A further object of the present invention is to provide a centrifugal pump for processing molten urea and a relative plant which is less complex than what is currently available.

A further object of the present invention is to provide a centrifugal pump for processing molten urea and a relative plant having less malfunction risks and lower wear of components.

Such objectives are achieved by the subject-matter of the independent claims.

According to a first aspect, the present invention relates to a centrifugal pump for processing molten urea according to claim 1.

According to a second aspect, the present invention relates to a plant for processing molten urea according to claim 9.

### LIST OF THE DRAWINGS

The present invention will be clearer from the following detailed description to be considered together with the accompanying drawings wherein:

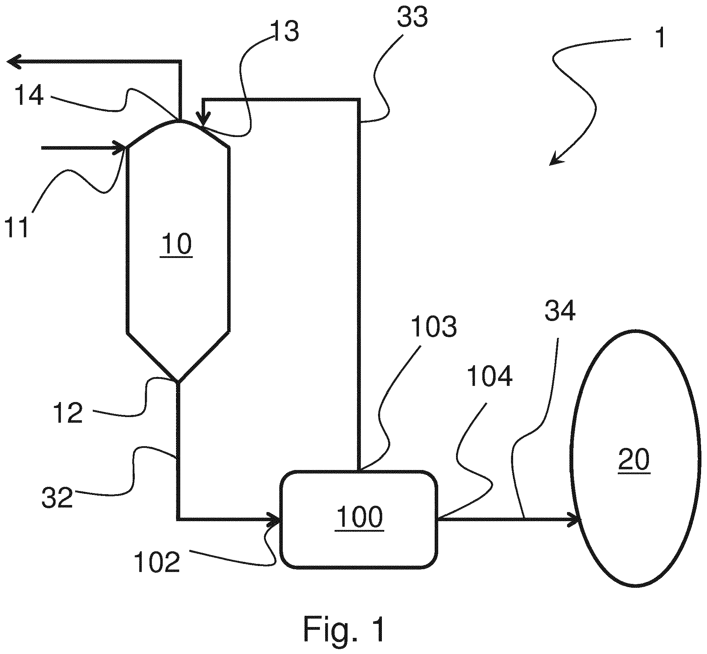

Fig. 1 shows a schematic view of a plant for processing molten urea according to the present invention;
Fig. 2 shows a schematic view of a centrifugal pump for processing molten urea according to the present invention;
Fig. 3 shows a schematic view of a detail of a plant for processing molten urea according to a possible embodiment of the present invention.
Fig. 4 shows a schematic view of a detail of a plant for processing molten urea with the possibility of internal circulation.
Fig.5 shows a schematic view of a detail of a plant for processing molten urea with flushing possibility.

As easily understandable, there are several modes to implement in practice the present invention which is defined in the accompanying claims and is not limited either by the following detailed description or by the accompanying drawings.

### DETAILED DESCRIPTION

With reference to the attached figures, a plant for processing molten urea, object of the present invention, is generically indicated with the reference number 1 and hereinafter reference to it will be made with the annotation "plant 1".

Plant 1 comprises a vacuum separator 10 for molten urea provided with a primary inlet 11, an outlet for molten urea 12, a recirculation inlet 13 and an outlet for gas 14. Preferably, the recirculation inlet 13 is arranged at or in the proximity of an upper end of the separator 10 and the outlet for molten urea 12 is arranged at or in the proximity of a lower end of the separator 10.

The plant 1 also comprises a centrifugal pump for processing molten urea, which forms a further object of the present invention and hereinafter it will be indicated with the reference number 100 and named as "centrifugal pump 100".

The centrifugal pump 100 has an inlet 102 and a delivery outlet 104. The inlet 102 corresponds to the suction of the centrifugal pump 100 and, when operated, is fluidly connected to the outlet for molten urea 12 of the separator 10 to receive molten urea from it through a suction duct 32. The delivery outlet 104 corresponds to the delivery of the centrifugal pump 100 and, when operated, is fluidly connectable to a reactor 20 for the production of melamine from urea, in order to feed it with a flow rate of molten urea through a delivery duct 34.

The centrifugal pump 100 is configured to receive molten urea at a preselected suction pressure at the inlet 102 and allow an output of molten urea at a delivery pressure at the delivery outlet 104, greater than the suction pressure. Preferably, the suction pressure is comprised between 0.5 bar and 5 bar, even more preferably between 1 bar and 2 bar, particularly of about 1.5 bar. Such suction pressure is determined by the greater height above ground level of the separator 10 (which operates under conditions of absolute vacuum or slight overpressure) with respect to the inlet 102. The difference in height between the separator 10 and the pump inlet 102 must therefore be such as to guarantee a positive pressure in any case.

The delivery pressure is preferably comprised between 60 bar and 100 bar, even more preferably between 70 bar and 90 bar, particularly of about 81 bar. Accordingly, the centrifugal pump 100 has a prevalence preferably comprised between 60 bar and 100 bar, even more preferably between 70 bar and 90 bar, particularly of about 80 bar.

Preferably, the centrifugal pump 100 is configured to process a flow rate of molten urea greater than 15 m³/h, even more preferably greater than 25 m³/h.

Preferably, the centrifugal pump 100 comprises a heating system configured to maintain an internal temperature of the molten urea greater than a preselected value, particularly greater than its melting temperature, equal to about 133 °C, in order to avoid solidification inside the centrifugal pump 100 itself.

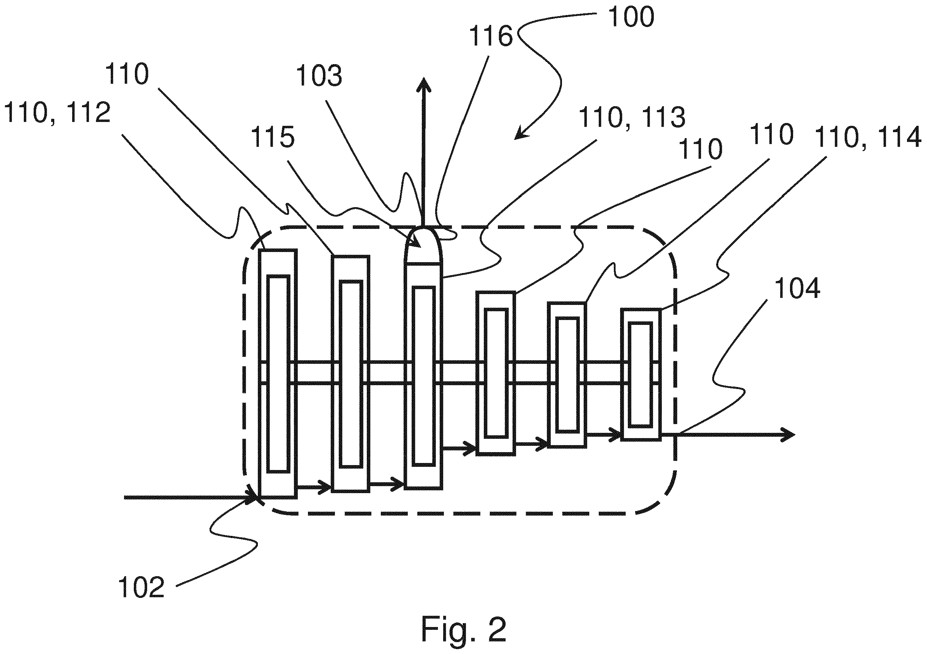

Preferably, the centrifugal pump 100 is multi-stage and comprises a plurality of centrifugal stages 110 serially arranged. Generally, each centrifugal stage 110 has a compression chamber and an impeller arranged inside it, as shown in Fig. 2. Particularly, the plurality of centrifugal stages 110 includes an initial stage 112 arranged at the inlet 102 to receive molten urea through it and a final stage 114 arranged at the delivery outlet 104 to send molten urea to the reactor 20 through it.

Between the initial stage 112 and the final stage 114 the pressure of the molten urea gradually increases passing through the various centrifugal stages 110.

The plurality of centrifugal stages 110 comprises an intermediate pressure stage 113, operatively serially arranged between the initial stage 112 and the final stage 114.

Particularly, the intermediate-pressure stage 113 has an intermediate outlet 103 configured and arranged to allow the output of molten urea from such stage at an intermediate pressure greater than the suction pressure and lower than the delivery pressure. Preferably, the intermediate pressure is comprised between 3 bar and 15 bar, even more preferably between 5 bar and 10 bar. In other words, the intermediate pressure is greater than the suction pressure by a value comprised between 2 bar and 14 bar, more preferably between 4 bar and 9 bar.

The value of said intermediate pressure depends on the serially arrangement of the intermediate-pressure stage 113 in the plurality of centrifugal stages 110. Preferably, the intermediate-pressure stage 113 is a centrifugal stage interposed between the initial stage 112 and the final stage 114. In some embodiments, the stage at intermediate pressure stage 113 can coincide with the initial stage 112.

Preferably, the intermediate outlet 103 is sized and configured to output a flow rate of molten urea comprised between 50% and 75% of the molten urea at the inlet, even more preferably between 60% and 70%, particularly about 67%.

Moreover, the intermediate outlet 103 when operated, is preferably located at an upper end of the intermediate-pressure stage 113 to allow a gas extraction or venting. The molten urea continuously generates reaction gases, particularly ammonia and carbon dioxide, and the intermediate-pressure stage 113 thus configured allows to convey the gases generated from urea during the movement through the intermediate outlet 103 with operating advantage of the subsequent compression stages.

Preferably, the intermediate-pressure stage 113 has a storage volume 115 arranged, an operative configuration, above the impeller, in fluid communication with the compression chamber. Particularly, the centrifugal pump 100 has a casing provided with a dome-shaped portion 116 vertically above the impeller of the intermediate-pressure stage 113 to define the above-mentioned storage volume 115. The intermediate outlet 103 is arranged at an upper end of the dome-shaped portion 116.

The storage volume 115 is configured to receive molten urea and gas from the compression chamber of the intermediate-pressure stage 113 during operation of the centrifugal pump 100 to allow its input through the intermediate outlet 103.

The intermediate outlet 103 is, when operated, fluidly connected to the separator 10 through a recirculation duct 33, such that to cause a recirculation stream of molten urea from the centrifugal pump 100 to the separator 10 such as to prevent accumulation of solid residues inside it. Particularly, the recirculation duct 33 is fluidly connected to the recirculation inlet 13 of the separator 10. The feed duct 32 is connected to the outlet for molten urea 12. This configuration allows to determine a continuous flow of molten urea from the recirculation inlet 13 to the outlet for molten urea 12 through the entire extension of the separator 10.

The plant 1 further comprises the reactor 20 for producing melamine fluidly connected to the delivery outlet 104 of the centrifugal pump 100 to receive from it pressurized molten urea through the delivery duct 34.

According to a possible embodiment, the centrifugal pump 100 has a variable speed to regulate the flow rate of the molten urea directed to the reactor 20.

According to a possible alternative embodiment, the centrifugal pump 100 has a fixed rotation speed and the plant 1 comprises a regulation valve arranged along the delivery duct 34, between the delivery outlet 104 of the centrifugal pump 100 and the reactor 20, to regulate the flow rate of the molten urea directed to the reactor 20.

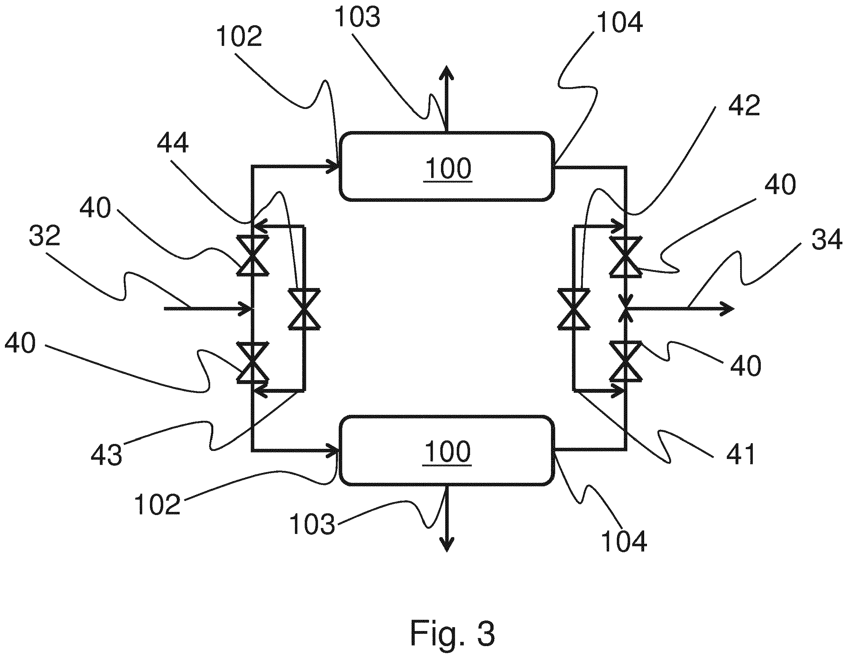

Preferably, plant 1 comprises two of the above-described centrifugal pumps 100, arranged in parallel for redundancy, as illustrated in Fig. 3, to compress the molten urea directed from the separator 10 to the reactor 20. One of the two centrifugal pumps 100 is configurable in stand-by while the other is running.

A plurality of valves 40 is arranged along the suction and delivery ducts of the two valves to allow the centrifugal pump 100, when operated, to be connected in line between the separator 10 and the reactor 20 and to isolate the other. Preferably, plant 1 further comprises a first flushing line 41 controlled by a related valve 42 and arranged to cause a fluid coupling between the delivery outlets 104 of the two centrifugal pumps 100, bypassing the valves 40. Moreover, plant 1 can comprise a second flushing line 43 controlled by a related valve 44 and arranged to cause a fluid coupling between the inlets 102 of the two centrifugal pumps 100, bypassing the valves 40.

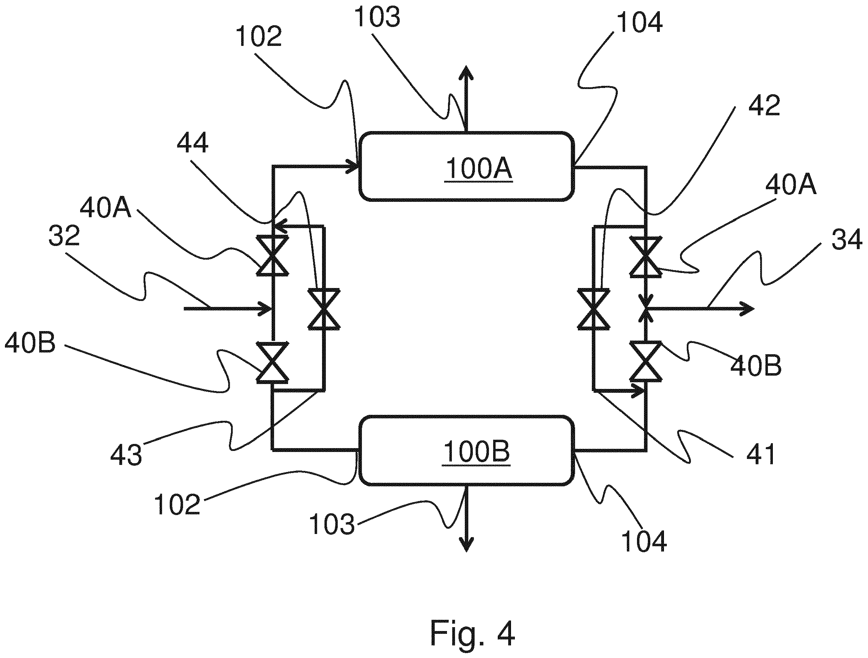

Fig. 4 shows an example of an operational configuration of the plant 1 wherein the centrifugal pump 100A is running, the centrifugal pump 100B is in stand-by, the valves 40A, 42 and 44 are open and the valves 40B are closed.

In the absence of the second flushing line 43, the re-circulation of the fluid in the centrifugal pump 100B in stand-by can be obtained by leaving open or partially open the valve 40B upstream (or in suction) of the centrifugal pump 100B in stand-by. Advantageously, the first and the second flushing line 41 and 43 allows to establish, during the operation of one of the two centrifugal pumps 100, a flow of molten urea from the delivery outlet 104 of the running centrifugal pump 100 to the inlet 102 of the other centrifugal pump 100 in stand-by, circulating inside the pump in stand-by, and reaching the suction line 102 of the running pump.

By means of this internal circulation in the stand-by pump it is avoided that molten urea can stay inside it with the possibility of decomposition and solidification.

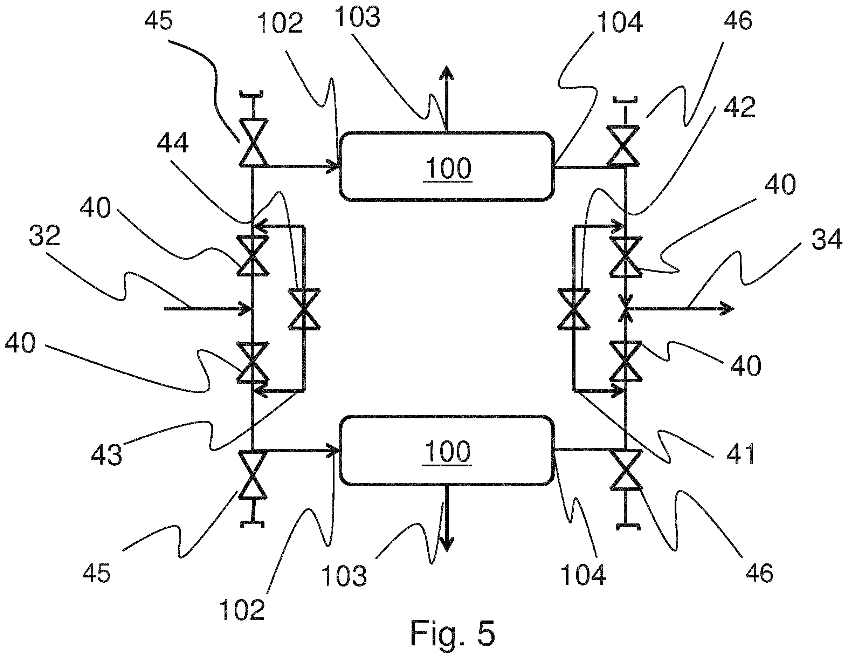

Preferably, plant 1 further comprises a system for washing the one or more centrifugal pumps 100 after their shutdown, an example of which is given in Fig. 5.

The washing of the centrifugal pumps 100 consists in emptying them from the molten urea by draining them into a recovery manifold, followed by flushing with steam and subsequently with water which are in turn recovered to avoid environmental pollution. For this purpose, plant 1 comprises a plurality of access portions 45 and 46, fluidly coupled to the centrifugal pumps 100 to allow their emptying and flushing. Particularly plant 1 comprises a prima access portion 45 upstream of the centrifugal pump 100 and a second access portion 46 downstream of each centrifugal pump 100. Preferably, the first and second access portions 45 e 46 consist of rapid connections equipped with valves, which allow the connection of drainage and / or flushing ducts to perform these operations.

These first and second access portions 45 and 46 allow access to any areas where the molten urea may have solidified, which tends to solidify in the dead points, i.e. without circulation and renewal of the product, for example the areas upstream of the rapid connection valves.

The described embodiments overcome the limitations of the prior art.

The use of the centrifugal pump with these characteristics in place of the traditional reciprocating pumps allows to process a greater amount of molten urea without having to adopt a large number of reciprocating heads or pumps in parallel.

The use of the centrifugal pump with these characteristics prevents the phenomena of cavitation in the suction and allows not to use additional pumps to pre-compress the molten urea.

The described centrifugal pump allows to send a recirculation flow rate at right pressure to the vacuum separator, if present, in order to prevent the accumulation of solid residues without requiring a dedicated pump.

The described centrifugal pump also allows to avoid problems caused to the plant by the delivery pulsating pressure of the reciprocating pumps.

The use of the described solutions makes it possible to create simpler plants having less malfunction risks and lower wear of components.

Comparing the construction and operating peculiarities of centrifugal pumps with respect to reciprocating pumps, the greater simplicity of washing the former compared to the latter is clear, considering that:
- there is only one pumping body and not a plurality of in parallel bodies with the possibility of by-passing by the washing fluid or urea retention during the drainage phase, which make the operation unsafe;
- they can be flushed in both directions, lacking in the internal check valves present in the reciprocating pumps.

## Claims

1. A centrifugal pump (100) for processing molten urea having:
- an inlet (102) adapted to receive molten urea at a suction pressure;
- a delivery outlet (104) adapted to allow an output of molten urea at a delivery pressure greater than said suction pressure;
wherein said centrifugal pump (100) comprises a heating system adapted to maintain a temperature of the molten urea inside the centrifugal pump (100) greater than a predetermined value,
the centrifugal pump being **characterised in that**
it has an intermediate outlet (103) adapted to allow an output of molten urea at an intermediate pressure greater than said suction pressure and lower than said delivery pressure.

2. The centrifugal pump (100) according to claim 1, comprising a plurality of centrifugal stages (110) arranged in series;
said plurality of centrifugal stages (110) comprising:
- an intermediate pressure stage (113) arranged and/or configured to cause a molten urea pressure equal to said intermediate pressure;
said intermediate outlet (103) being arranged at said intermediate pressure stage (113) to receive molten urea from said intermediate pressure stage (113).

3. The centrifugal pump (100) according to claim 2, wherein said plurality of centrifugal stages (110) comprises:
- an initial stage (112) arranged at said inlet (102) to receive molten urea from said inlet (102);
- a final stage (114) arranged at said delivery outlet (104) to send molten urea to said delivery outlet (104);
said intermediate pressure stage (113) being operatively arranged between said initial stage (112) and said final stage (114).

4. The centrifugal pump (100) according to claim 2 or 3, wherein said intermediate outlet (103) is arranged at an upper end of said intermediate pressure stage (113) to allow a gas extraction from said intermediate pressure stage (113) through said intermediate outlet (103).

5. The centrifugal pump (100) according to claim 4, wherein said intermediate pressure stage (113) has a compression chamber, an impeller arranged in said compression chamber and a storage volume (115) arranged above said impeller, in fluid communication with said compression chamber; said storage volume (115) being configured to receive molten urea and gas from said compression chamber during an operation of the centrifugal pump (100) and said intermediate outlet (103) being arranged at an upper end of said storage volume (115).

6. The centrifugal pump (100) according to claim 5, comprising a casing provided with a dome-shaped portion (116), said dome-shaped portion (116) defining superiorly said storage volume (115).

7. The centrifugal pump (100) according to one or more of the preceding claims, adapted to process a flow rate of molten urea greater than 15 m³/h, preferably greater than 25m³/h, wherein said delivery pressure is greater than said suction pressure by a value comprised between 50 bar and 100 bar, preferably between 70 bar and 90 bar.

8. The centrifugal pump (100) according to one or more of the preceding claims, wherein said intermediate pressure is greater than said suction pressure by a value comprised between 2 bar and 14 bar, preferably between 4 bar and 9 bar, and wherein said centrifugal pump (100) is configured to direct a flow rate of molten urea comprised between 50% and 75% of the molten urea at the inlet, preferably between 60% and 70%, through said intermediate outlet (103).

9. A plant (1) for processing molten urea, in particular for producing melamine with a high-pressure process, comprising:
- a separator (10) for molten urea having a primary inlet (11), a recirculation inlet (13) and an outlet for molten urea (12);
- at least one centrifugal pump (100) having:
- an inlet (102) adapted to receive molten urea at a suction pressure;
- a delivery outlet (104) adapted to allow an output of molten urea at a delivery pressure greater than said suction pressure;
- an intermediate outlet (103) adapted to allow an output of molten urea at an intermediate pressure greater than said suction pressure and lower than said delivery pressure;
wherein the inlet (102) of the centrifugal pump (100) is fluidly connected to the outlet for molten urea (12) of the separator (10) to receive molten urea at the inlet coming from said separator (10);
wherein the recirculation inlet (13) of the separator (10) is fluidly connected to the intermediate outlet (103) of the centrifugal pump (100) to receive recirculation molten urea at the inlet coming from said centrifugal pump (100).

10. The plant according to claim 9, wherein the centrifugal pump (100) is according to one or more of claims 1 to 8.

11. The plant (1) according to claim 9 or 10, comprising a reactor (20) for producing melamine in a high-pressure process, fluidly connected to the delivery outlet (104) of said centrifugal pump (100) to receive pressurized molten urea from said centrifugal pump (100).

12. The plant (1) according to claim 11, wherein
said centrifugal pump (100) has a variable speed to regulate a flow rate and/or a pressure of the molten urea directed to said reactor (20); or
said centrifugal pump (100) has a fixed rotation speed and said plant (1) comprises a regulation valve operatively arranged between the delivery outlet (104) of said centrifugal pump (100) and said reactor (20) to regulate a flow rate and/or a pressure of the molten urea directed to said reactor (20).

13. The plant (1) according to one or more of claims 9 to 12, comprising two centrifugal pumps (100) according to the pump features of claim 9,
said two centrifugal pumps (100) being arranged in parallel;
one of said two centrifugal pumps (100) being configurable in stand-by while the other is running.

14. The plant (1) according to claim 13, comprising a first flushing line (41) adapted to cause a fluid coupling between the delivery outlets (104) of said two centrifugal pumps (100) and a second flushing line (43) adapted to cause a fluid coupling between the inlets (102) of said two centrifugal pumps (100);
the running centrifugal pump (100) being adapted to cause a flow of molten urea from the delivery outlet (104) to the inlet (102) of said centrifugal pump (100) in stand-by through said first and second flushing line (41, 43) in an operating condition of said plant (1).

15. The plant (1) according to one or more of claims 9 to 14, comprising at least one first access portion (45) fluidly coupled to said at least one centrifugal pump (100) upstream of said at least one centrifugal pump (100) and at least one second access portion (46) fluidly coupled to said at least one centrifugal pump (100) downstream of said at least one centrifugal pump (100);
said first and second access portions (45, 46) being adapted to drain and/or flush said at least one centrifugal pump (100).

## Patentansprüche

1. Kreiselpumpe (100) zur Verarbeitung von geschmolzenem Harnstoff, aufweisend:
- einen Einlass (102), der geeignet ist, geschmolzenen Harnstoff mit einem Ansaugdruck aufzunehmen;
- einen Abgabeauslass (104), der geeignet ist, eine Abgabe von geschmolzenem Harnstoff bei einem Abgabedruck zu ermöglichen, der größer ist als der Ansaugdruck;
wobei die Kreiselpumpe (100) ein Heizsystem umfasst, das geeignet ist, eine Temperatur des geschmolzenen Harnstoffs innerhalb der Kreiselpumpe (100) über einem vorbestimmten Wert zu halten, wobei die Kreiselpumpe **dadurch gekennzeichnet ist, dass** sie einen Zwischenauslass (103) aufweist, der geeignet ist, eine Abgabe von geschmolzenem Harnstoff bei einem Zwischendruck zu ermöglichen, der größer als der Ansaugdruck und kleiner als der Abgabedruck ist.

2. Kreiselpumpe (100) nach Anspruch 1, umfassend eine Vielzahl von in Reihe angeordneten Kreiselstufen (110);
wobei die Vielzahl von Kreiselstufen (110) umfasst:
- eine Zwischendruckstufe (113), die so angeordnet und/oder konfiguriert ist, dass sie einen Druck des geschmolzenen Harnstoffs erzeugt, der dem Zwischendruck entspricht;
wobei der Zwischenauslass (103) an der Zwischendruckstufe (113) angeordnet ist, um geschmolzenen Harnstoff von der Zwischendruckstufe (113) aufzunehmen.

3. Kreiselpumpe (100) nach Anspruch 2, wobei die Vielzahl von Kreiselstufen (110) umfasst:
- eine Anfangsstufe (112), die an dem Einlass (102) angeordnet ist, um geschmolzenen Harnstoff von dem Einlass (102) aufzunehmen;
- eine Endstufe (114), die an dem Abgabeauslass (104) angeordnet ist, um geschmolzenen Harnstoff zu dem Abgabeauslass (104) zu leiten;
wobei die Zwischendruckstufe (113) operativ zwischen der Anfangsstufe (112) und der Endstufe (114) angeordnet ist.

4. Kreiselpumpe (100) nach Anspruch 2 oder 3, wobei der Zwischenauslass (103) an einem oberen Ende der Zwischendruckstufe (113) angeordnet ist, um eine Gasentnahme aus der Zwischendruckstufe (113) durch den Zwischenauslass (103) zu ermöglichen.

5. Kreiselpumpe (100) nach Anspruch 4, wobei die Zwischendruckstufe (113) eine Verdichtungskammer, ein in der Verdichtungskammer angeordnetes Laufrad und ein oberhalb des Laufrades angeordnetes Speichervolumen (115) aufweist, das in Fluidverbindung mit der Verdichtungskammer steht;
wobei das Speichervolumen (115) so konfiguriert ist, dass es geschmolzenen Harnstoff und Gas aus der Verdichtungskammer während eines Betriebs der Kreiselpumpe (100) aufnimmt, und wobei der Zwischenauslass (103) an einem oberen Ende des Speichervolumens (115) angeordnet ist.

6. Kreiselpumpe (100) nach Anspruch 5, umfassend eine Karkasse, die mit einem kuppelförmigen Abschnitt (116) versehen ist, wobei der kuppelförmige Abschnitt (116) das Speichervolumen (115) nach oben hin begrenzt.

7. Kreiselpumpe (100) nach einem oder mehreren der vorstehenden Ansprüche, die zur Verarbeitung einer Durchflussmenge von geschmolzenem Harnstoff von mehr als 15 m³/h, vorzugsweise mehr als 25 m³/h, geeignet ist, wobei der Abgabedruck um einen Wert zwischen 50 bar und 100 bar, vorzugsweise zwischen 70 bar und 90 bar, größer ist als der Ansaugdruck.

8. Kreiselpumpe (100) nach einem oder mehreren der vorstehenden Ansprüche, wobei der Zwischendruck um einen Wert zwischen 2 bar und 14 bar, vorzugsweise zwischen 4 bar und 9 bar, größer ist als der Ansaugdruck und wobei die Kreiselpumpe (100) so konfiguriert ist, dass sie eine Durchflussmenge von geschmolzenem Harnstoff, die zwischen 50 % und 75 % des geschmolzenen Harnstoffs am Einlass, vorzugsweise zwischen 60 % und 70 %, liegt, durch den Zwischenauslass (103) leitet.

9. Anlage (1) zur Verarbeitung von geschmolzenem Harnstoff, insbesondere zur Herstellung von Melamin in einem Hochdruckverfahren, umfassend:
- einen Separator (10) für geschmolzenen Harnstoff, aufweisend einen Primäreinlass (11), einen Rezirkulationseinlass (13) und einen Auslass für geschmolzenen Harnstoff (12);
- mindestens eine Kreiselpumpe (100), aufweisend:
- einen Einlass (102), der geeignet ist, geschmolzenen Harnstoff mit einem Ansaugdruck aufzunehmen;
- einen Abgabeauslass (104), der geeignet ist, eine Abgabe von geschmolzenem Harnstoff bei einem Abgabedruck zu ermöglichen, der größer ist als der Ansaugdruck;
- einen Zwischenauslass (103), der eine Abgabe von geschmolzenem Harnstoff bei einem Zwischendruck ermöglicht, der größer als der Ansaugdruck und kleiner als der Abgabedruck ist; wobei der Einlass (102) der Kreiselpumpe (100) fluidisch mit dem Auslass für geschmolzenen Harnstoff (12) des Separators (10) verbunden ist, um geschmolzenen Harnstoff am Einlass, der von dem Separator (10) kommt, aufzunehmen;
wobei der Rezirkulationseinlass (13) des Separators (10) fluidisch mit dem Zwischenauslass (103) der Kreiselpumpe (100) verbunden ist, um geschmolzenen Harnstoff am Einlass, der von der Kreiselpumpe (100) kommt, in Rezirkulation aufzunehmen.

10. Anlage nach Anspruch 9, wobei die Kreiselpumpe (100) nach einem oder mehreren der Ansprüche 1 bis 8 ausgeführt ist.

11. Anlage (1) nach Anspruch 9 oder 10, umfassend einen Reaktor (20) zur Herstellung von Melamin in einem Hochdruckverfahren, der mit dem Abgabeauslass (104) der Kreiselpumpe (100) fluidisch verbunden ist, um unter Druck stehenden geschmolzenen Harnstoff von der Kreiselpumpe (100) aufzunehmen.

12. Anlage (1) nach Anspruch 11, wobei
die Kreiselpumpe (100) eine variable Geschwindigkeit aufweist, um eine Durchflussmenge und/oder einen Druck des geschmolzenen Harnstoffs, der zu dem Reaktor (20) geleitet wird, zu regulieren; oder die Kreiselpumpe (100) eine feste Rotationsgeschwindigkeit aufweist und die Anlage (1) ein Regulierventil umfasst, das operativ zwischen dem Abgabeauslass (104) der Kreiselpumpe (100) und dem Reaktor (20) angeordnet ist, um eine Durchflussmenge und/oder einen Druck des geschmolzenen Harnstoffs, der zu dem Reaktor (20) geleitet wird, zu regulieren.

13. Anlage (1) nach einem oder mehreren der Ansprüche 9 bis 12, umfassend zwei Kreiselpumpen (100) gemäß den Pumpenmerkmalen von Anspruch 9, wobei die beiden Kreiselpumpen (100) parallel angeordnet sind;
wobei eine der beiden Kreiselpumpen (100) als Stand-by-Pumpe konfiguriert werden kann, während die andere in Betrieb ist.

14. Anlage (1) nach Anspruch 13, umfassend eine erste Spülleitung (41), die geeignet ist, eine Fluidkopplung zwischen den Abgabeauslässen (104) der beiden Kreiselpumpen (100) herzustellen, und eine zweite Spülleitung (43), die geeignet ist, eine Fluidkopplung zwischen den Einlässen (102) der beiden Kreiselpumpen (100) herzustellen;
wobei die laufende Kreiselpumpe (100) so ausgelegt ist, dass sie in einem Betriebszustand der Anlage (1) einen Fluss von geschmolzenem Harnstoff vom Abgabeauslass (104) zum Einlass (102) der Stand-by-Kreiselpumpe (100) durch die erste und zweite Spülleitung (41, 43) bewirkt.

15. Anlage (1) nach einem oder mehreren der Ansprüche 9 bis 14, umfassend mindestens einen ersten Zugangsabschnitt (45), der mit der mindestens einen Kreiselpumpe (100) stromaufwärts der mindestens einen Kreiselpumpe (100) fluidisch gekoppelt ist, und mindestens einen zweiten Zugangsabschnitt (46), der mit der mindestens einen Kreiselpumpe (100) stromabwärts der mindestens einen Kreiselpumpe (100) fluidisch gekoppelt ist;
wobei die ersten und zweiten Zugangsabschnitte (45, 46) zum Entleeren und/oder Spülen der mindestens einen Kreiselpumpe (100) geeignet sind.

## Revendications

1. Pompe centrifuge (100) pour le traitement de l'urée fondue ayant :
- une entrée (102) adaptée pour recevoir de l'urée fondue à une pression d'aspiration ;
- une sortie de refoulement (104) adaptée pour permettre une sortie d'urée fondue à une pression de refoulement supérieure à ladite pression d'aspiration ;
dans laquelle ladite pompe centrifuge (100) comprend un système de chauffage adapté pour maintenir une température de l'urée fondue à l'intérieur de la pompe centrifuge (100) supérieure à une valeur prédéterminée,
la pompe centrifuge étant **caractérisée en ce qu'**elle a une sortie intermédiaire (103) adaptée pour permettre une sortie d'urée fondue à une pression intermédiaire supérieure à ladite pression d'aspiration et inférieure à ladite pression de refoulement.

2. Pompe centrifuge (100) selon la revendication 1, comprenant une pluralité d'étages centrifuges (110) disposés en série ;
ladite pluralité d'étages centrifuges (110) comprenant :
- un étage de pression intermédiaire (113) disposé et/ou configuré pour provoquer une pression d'urée fondue égale à ladite pression intermédiaire ;
ladite sortie intermédiaire (103) étant disposée audit étage de pression intermédiaire (113) pour recevoir de l'urée fondue provenant de l'étage de pression intermédiaire (113).

3. Pompe centrifuge (100) selon la revendication 2, dans laquelle ladite pluralité d'étages centrifuges (110) comprend :
- un étage initial (112) disposé à ladite entrée (102) pour recevoir de l'urée fondue de ladite entrée (102) ;
- un étage final (114) disposé à ladite sortie de refoulement (104) pour envoyer de l'urée fondue à ladite sortie de refoulement (104) ; ledit étage de pression intermédiaire (113) étant disposé de manière opérationnelle entre ledit étage initial (112) et ledit étage final (114).

4. Pompe centrifuge (100) selon la revendication 2 ou 3, dans laquelle ladite sortie intermédiaire (103) est disposée à une extrémité supérieure dudit étage de pression intermédiaire (113) pour permettre une extraction de gaz dudit étage de pression intermédiaire (113) à travers ladite sortie intermédiaire (103).

5. Pompe centrifuge (100) selon la revendication 4, dans laquelle ledit étage de pression intermédiaire (113) a une chambre de compression, une roue disposée dans ladite chambre de compression et un volume de stockage (115) disposé au-dessus de ladite roue, en communication fluidique avec ladite chambre de compression ;
ledit volume de stockage (115) étant configuré pour recevoir de l'urée fondue et du gaz provenant de ladite chambre de compression pendant le fonctionnement de la pompe centrifuge (100) et ladite sortie intermédiaire (103) étant disposée à une extrémité supérieure dudit volume de stockage (115).

6. Pompe centrifuge (100) selon la revendication 5, comprenant un boîtier pourvu d'une partie en forme de dôme (116), ladite partie en forme de dôme (116) définissant supérieurement ledit volume de stockage (115).

7. Pompe centrifuge (100) selon l'une ou plusieurs des revendications précédentes, adaptée pour traiter un débit d'urée fondue supérieur à 15 m³/h, de préférence supérieur à 25 m³/h, dans laquelle ladite pression de refoulement est supérieure à ladite pression d'aspiration d'une valeur comprise entre 50 bar et 100 bar, de préférence entre 70 bar et 90 bar.

8. Pompe centrifuge (100) selon l'une ou plusieurs des revendications précédentes, dans laquelle ladite pression intermédiaire est supérieure à ladite pression d'aspiration d'une valeur comprise entre 2 bar et 14 bar, de préférence entre 4 bar et 9 bar, et dans laquelle ladite pompe centrifuge (100) est configurée pour diriger un débit d'urée fondue compris entre 50 % et 75 % de l'urée fondue à l'entrée, de préférence entre 60 % et 70 %, à travers ladite sortie intermédiaire (103).

9. Installation (1) pour le traitement de l'urée fondue, en particulier pour la production de mélamine par un procédé à haute pression, comprenant :
- un séparateur (10) d'urée fondue ayant une entrée primaire (11), une entrée de recirculation (13) et une sortie d'urée fondue (12) ;
- au moins une pompe centrifuge (100) ayant :
- une entrée (102) adaptée pour recevoir de l'urée fondue à une pression d'aspiration ;
- une sortie de refoulement (104) adaptée pour permettre une sortie d'urée fondue à une pression de refoulement supérieure à ladite pression d'aspiration ;
- une sortie intermédiaire (103) adaptée pour permettre une sortie d'urée fondue à une pression intermédiaire supérieure à ladite pression d'aspiration et inférieure à ladite pression de refoulement ; dans laquelle l'entrée (102) de la pompe centrifuge (100) est reliée fluidiquement à la sortie d'urée fondue (12) du séparateur (10) pour recevoir de l'urée fondue à l'entrée provenant dudit séparateur (10) ; dans laquelle l'entrée de recirculation (13) du séparateur (10) est reliée fluidiquement à la sortie intermédiaire (103) de la pompe centrifuge (100) pour recevoir de l'urée fondue de recirculation à l'entrée provenant de ladite pompe centrifuge (100).

10. Installation selon la revendication 9, dans laquelle la pompe centrifuge (100) est selon l'une ou plusieurs des revendications 1 à 8.

11. Installation (1) selon la revendication 9 ou 10, comprenant un réacteur (20) pour la production de mélamine dans un processus à haute pression, relié fluidiquement à la sortie de refoulement (104) de ladite pompe centrifuge (100) pour recevoir de l'urée fondue sous pression provenant de ladite pompe centrifuge (100).

12. Installation (1) selon la revendication 11, dans laquelle ladite pompe centrifuge (100) a une vitesse variable pour réguler un débit et/ou une pression de l'urée fondue dirigée vers ledit réacteur (20) ; ou ladite pompe centrifuge (100) a une vitesse de rotation fixe et ladite installation (1) comprend une vanne de régulation fonctionnellement disposée entre la sortie de refoulement (104) de ladite pompe centrifuge (100) et dudit réacteur (20) pour réguler un débit et/ou une pression de l'urée fondue dirigée vers ledit réacteur (20).

13. Installation (1) selon une ou plusieurs des revendications 9 à 12, comprenant deux pompes centrifuges (100) selon les caractéristiques de pompe selon la revendication 9, lesdites deux pompes centrifuges (100) étant disposées en parallèle ;
l'une desdites deux pompes centrifuges (100) pouvant être configurée en veille tandis que l'autre est en marche.

14. Installation (1) selon la revendication 13, comprenant une première conduite de rinçage (41) adaptée pour provoquer un couplage fluidique entre les sorties de refoulement (104) desdites deux pompes centrifuges (100) et une seconde conduite de rinçage (43) adaptée pour provoquer un couplage fluidique entre les entrées (102) desdites deux pompes centrifuges (100) ;
la pompe centrifuge en marche (100) étant adaptée pour provoquer un écoulement d'urée fondue de la sortie de refoulement (104) à l'entrée (102) de ladite pompe centrifuge (100) en veille à travers lesdites première et seconde conduites de rinçage (41, 43) dans un état de fonctionnement de ladite installation (1).

15. Installation (1) selon une ou plusieurs des revendications 9 à 14, comprenant au moins une première partie d'accès (45) couplée fluidiquement à ladite au moins une pompe centrifuge (100) en amont de ladite au moins une pompe centrifuge (100) et au moins une seconde partie d'accès (46) couplée fluidiquement à ladite au moins une pompe centrifuge (100) en aval de ladite au moins une pompe centrifuge (100) ;
lesdites première et seconde parties d'accès (45, 46) étant adaptées pour drainer et/ou purger ladite au moins une pompe centrifuge (100).
